(19) Europäisches Patentamt
European Patent Office
Office européen des brevets

(11) **EP 1 739 620 B1**

(12) **EUROPEAN PATENT SPECIFICATION**

(45) Date of publication and mention
of the grant of the patent:
**24.12.2008 Bulletin 2008/52**

(51) Int Cl.:
***G06T 5/00*** *(2006.01)*

(21) Application number: **05105782.6**

(22) Date of filing: **29.06.2005**

(54) **Method of identifying disturbing frequencies originating from the presence of an anti-scatter grid during acquisition of a radiation image.**

Methode zur Identifikation störender Frequenzen, die durch ein Streustrahlenraster bei der Aufnahme eines radiographischen Bildes entstehen

Méthode d'identifier des fréquences inquiétantes provenant de la présence d'une grille anti-éclatement pendant la saisie d'une image de rayonnement

(84) Designated Contracting States:
**BE DE FR GB NL**

(43) Date of publication of application:
**03.01.2007 Bulletin 2007/01**

(73) Proprietor: **Agfa HealthCare NV**
**2640 Mortsel (BE)**

(72) Inventor: **Behiels, Gert**
**c/o AGFA-GEVAERT**
**2640 Mortsel (BE)**

(74) Representative: **Theunis, Patrick et al**
**Agfa-Gevaert N.V.**
**HE / Intellectual Property 3802**
**Septestraat 27**
**2640 Mortsel (BE)**

(56) References cited:
**EP-A- 1 505 540          US-B1- 6 269 176**

**Description**

FIELD OF THE INVENTION

The present invention relates to method of identifying disturbing frequencies originating from the use of an anti scatter grid during radiation image recording in computed radiography.

BACKGROUND OF THE INVENTION

A commonly used technique to reduce the amount of scattered X-rays in computed radiography, digital radiography as well as classical film-based X-ray systems is the use of anti-scatter grids. These grids are lead foil strips, placed apart at a certain distance in a suitable covering.
There exist different types of anti-scatter grids. In parallel grids, the lead foil strips are parallel, while in honeycomb grids the strips are placed in a honeycomb pattern. The grids are stationary or moving. The use of these grids effectively reduces the radiation scattering but occasionally introduces artifacts such as grid lines into the image.

In a moving system, the motion of the grids removes the grid lines in the image. However, in some circumstances e.g. short exposure time or malfunctioning of the system, the artifacts remain in the image. With stationary grids, the grid lines are almost always visible.

If the image is formed digitally or converted afterwards to a digital image representation, Moiré artifacts may appear when displaying the image at a certain scale. These low frequent Moiré artifacts are mostly disturbing and should be eliminated. Before displaying the image, the grid lines, if present in the image, should be removed.

US patent US6,269,176 discloses a grid removal method for radiographic images, in which the grid is modelled - based on an estimate of the grid frequency obtained from the actual image - and then subtracted from the radiographic image. European patent application EP1,505,540 relates to a grid removal process in which the radiographic image is analyzed to determine the grid frequency which are then used to construct the grid removal filter in the spatial domain.

Current gridline correction techniques, when having detected a grid, suppress only the fundamental frequency in the image sometimes in combination with frequencies in the neighborhood of the harmonic frequencies. The assumption of the harmonic frequencies is not always true. The number of harmonic frequencies to be suppressed is fixed in the prior art methods.

It is an object of the present invention to provide a method to determine the disturbing frequencies in a signal representation of a radiation image that overcomes the above-mentioned shortcoming of the prior art.

SUMMARY OF THE INVENTION

The above-mentioned aspects are realised by a method as set out in claim 1.
Specific features for preferred embodiments of the invention are set out in the dependent claims.
Further advantages and embodiments of the present invention will become apparent from the following description and drawings.

The present invention determines which frequencies to suppress in an image signal representing an image originating from an x ray exposure whereby an anti scatter grid has been used in order to remove the annoying Moiré artifacts.

Having found the fundamental frequency of the anti scatter grid in a signal representation of an x ray image, the digitization of an image containing only an anti scatter grid is simulated. Next, spectrum analysis, e.g. Fourier analysis of this simulated digitized signal is used to determine which frequencies the image might be disturbed by the presence of the anti scatter grid. If the candidate frequencies are also present in the image, they are selected as frequencies to suppress.

BRIEF DESCRIPTION OF THE DRAWINGS

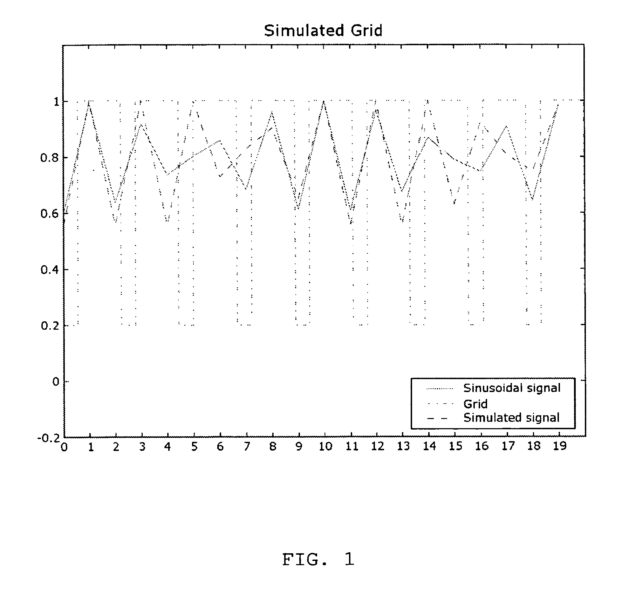

Fig. 1 shows the expected signal representation of a grid image at the pixel positions when only the fundamental frequency is taken into account (solid line). The dash-dotted line is the signal representation of a simulated grid, drawn at a higher resolution than the scanning resolution (pixel positions) of a read out device used for reading out a stored radiation image.
The dotted line shows the simulated signal after simulation of the digitization process of the digitizer. It is clear that the simulated signal contains more frequencies than only the fundamental frequency.

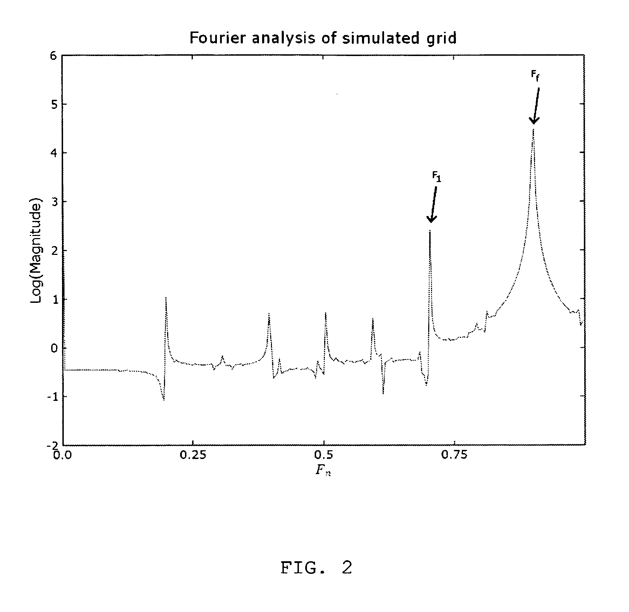

Fig. 2 shows the result of a Fourier analysis of the simulated grid signal. If we want to suppress the frequencies for which the log magnitude is greater than 2, the relative frequency $F_1$, at about 70% of the Nyquist frequency, is a candidate frequency for suppression. $F_f$ is the fundamental frequency found in the image of the grid.

DETAILED DESCRIPTION OF THE INVENTION

A specific embodiment of the method of the present invention will be described hereinafter.

The input of the method is the fundamental frequency or period of the anti-scatter grid in an x-ray image of the grid.

In the context of the present invention the x-ray image of the grid is obtained by means of a computed radiography system. The grid is irradiated by means of x-rays. The radiation image of the grid is stored in a radiation detector such as a photostimulable phosphor screen. The image is then read out in a read out device (also called digitizer) by scanning the screen with stimulating radiation. Image-wise modulated light emitted by the exposed screen upon stimulation is detected and converted into a signal representation.

The signal representation is analysed and the frequency at which the signal of the anti-scatter grid has maximum amplitude, hereinafter called the fundamental frequency $F_f$, with corresponding period $P_f$, is detected. This fundamental frequency does not necessarily coincide with the true grid frequency $F_g$, with corresponding period $P_g$.

If $F_s$ is the sampling frequency of the digitizer and $F_n$ the corresponding Nyquist frequency

$$F_n = \frac{F_s}{2} \; ,$$

one could try to guess the true grid frequency $F_g$. Having found this true grid frequency, the corresponding h$^{th}$ harmonic frequency to suppress in the image signal is

$$\left|F_h\right| = \left|k \cdot F_s - h \cdot F_g\right| \; ,$$

**Equation 1**

where k is chosen to satisfy

$$\left|F_h\right| \leq F_n \; .$$

The success of this scheme lies entirely in the exact guess of $F_g$ and the selection of enough harmonic frequencies to suppress in the image signal. Because we do not know how many frequencies to suppress, a different approach is preferably chosen.

Having detected the fundamental frequency $F_f$ in the signal representation of the radiation image of the grid, a grid is simulated.

For this purpose a signal representation is generated representing a grid with the distance between two grid lamella's being equal to $P_f$. This grid signal representation g is constructed at a higher resolution than the sampling frequency $F_s$ of the read out apparatus:

$$g_i = \begin{cases} a : 0 \leq i \bmod P_g < r \\ 1 : r \leq i \bmod P_g < P_g \end{cases}$$

$$\begin{cases} 0 \leq a < 1 \\ 0 \leq r < 1 \end{cases}$$

If the pixel size $P_s$ is for example 100$\mu$m, the grid is constructed at a resolution where one sample $i$ represents 1$\mu$m,
a is the attenuation of the radiation at the positions of the grid lamella's and r is the ratio of the lamella width to the distance between two lamella's.

This grid signal representation is then averaged over distance $P_s$ to obtain the simulated grid signal representation $G$ :

$$G_j = \sum_{j=P_s}^{(i+1)P_s - 1} g_j \; .$$

This averaging simulates the digitization process of the image read out device.

More complex simulations may be used, such as averaging the grid after convolution with a Gaussian kernel to simulate the flying spot of the light stimulation in some digitizers.
For most purposes, a simple averaging of the grid at the high resolution is sufficient.

Fourier analysis of the simulated grid representation $G$ shows at which frequencies the original image signal is disturbed (see figure 2).

Only the frequencies $F_h$, with an amplitude greater than a fixed threshold, are selected.
In the example of figure 2, in addition to the fundamental frequency $F_f$ located at about 0.9 times the Nyquist frequency, we also want to suppress the frequency located at about 0.7 times the Nyquist frequency.

The frequencies to be suppressed can be stored in a look up table with the fundamental frequency as the indexing variable and retrieved when these frequencies are to be suppressed in order to eliminate the disturbing effect in an image due to the presence of an anti-scatter grid at image recording.

The entries of the look up table can thus be obtained by the steps described higher, alternatively they can be obtained by image analysis, e.g. by applying equation (1).

**Claims**

1. A method of determining disturbing signal frequencies in a signal representation of radiation image originating from the presence of an anti-scatter grid during image acquisition, said signal representation being generated by an image read out and digitizing process, the method comprising the steps of

- generating an image of a simulated anti-scatter grid
- simulating the digitizing process by averaging said image of a simulated anti-scatter grid thereby generating a digitized simulated anti-scatter grid signal,
- analyzing said digitized simulated anti-scatter grid signal to determine said disturbing frequencies.

2. A method according to claim 1 wherein said analyzing step comprises applying a Fourier transformation to said digitized, simulated anti-scatter grid signal.

3. A method according to claim 1 wherein the frequency of the simulated anti-scatter grid equals the frequency at which the signal of said anti-scatter grid has maximum amplitude

4. A method according to claim 1 wherein the frequency of the simulated anti-scatter grid equals the actual frequency of said anti-scatter grid.

5. A method according to claim 1 wherein the frequency of the simulated anti-scatter grid equals an estimated grid frequency.

6. A method according to claim 1 wherein said disturbing frequencies resulting from said analyzing step which are prominently present in said signal representation of said radiation image are stored to be suppressed.

7. A method according to claim 1 wherein said disturbing frequencies are stored in a look up table.

**Patentansprüche**

1. Ein Verfahren zur Ermittlung von durch Anwesenheit eines Streustrahlenrasters während der Bilderfassung verursachten störenden Signalfrequenzen in einer Signaldarstellung eines Strahlenbildes, wobei die Signaldarstellung durch einen Prozess, der das Auslesen und Digitalisieren eines Bildes umfasst, erzeugt wird, wobei das Verfahren durch folgende Schritte **gekennzeichnet** ist :

   - Erzeugen eines Bildes eines simulierten Streustrahlenrasters,
   - Simulieren des Digitalisiervorgangs durch Mittelung des Bildes eines simulierten Streustrahlenrasters, wobei ein digitalisiertes Streustrahlenrastersignal erzeugt wird, und
   - Analysieren des digitalisierten simulierten Streustrahlenrastersignals zur Ermittlung der störenden Frequenzen.

2. Verfahren nach Anspruch 1, **dadurch gekennzeichnet, dass** im Analysierschritt eine Fourier-Transformation auf das digitalisierte simulierte Streustrahlenrastersignal durchgeführt wird.

3. Verfahren nach Anspruch 1, **dadurch gekennzeichnet, dass** die Frequenz des simulierten Streustrahlenrasters der Frequenz, bei der das Signal des Streustrahlenrasters eine maximale Amplitude aufweist, entspricht.

4. Verfahren nach Anspruch 1, **dadurch gekennzeichnet, dass** die Frequenz des simulierten Streustrahlenrasters der Istfrequenz des Streustrahlenrasters entspricht.

5. Verfahren nach Anspruch 1, **dadurch gekennzeichnet, dass** die Frequenz des simulierten Streustrahlenrasters einer geschätzten Rasterfrequenz entspricht.

6. Verfahren nach Anspruch 1, **dadurch gekennzeichnet, dass** die im Analysierschritt ermittelten störenden Frequenzen, die in bedeutendem Maße in der Signaldarstellung des Strahlenbildes enthalten sind, zwecks ihrer nachträglichen Löschung gespeichert werden.

7. Verfahren nach Anspruch 1, **dadurch gekennzeichnet, dass** die störenden Frequenzen in einer Nachschlagetabelle gespeichert werden.

**Revendications**

1. Procédé pour déterminer des fréquences de signaux parasites dans une représentation sous forme de signal d'une image radiographique, émanant de la présence d'une grille d'antidiffusion au cours de l'acquisition d'image, ladite représentation sous forme de signal étant générée par un processus de lecture et d'image et de numérisation d'image, le procédé comprenant les étapes consistant à :

   - générer une image d'une grille d'antidiffusion simulée ;
   - simuler le processus de numérisation en faisant la moyenne de ladite image d'une grille d'antidiffusion simulée, en générant ainsi un signal de grille d'antidiffusion simulée numérisée ;
   - analyser ledit signal de grille d'antidiffusion simulée numérisée afin de déterminer les fréquences parasites.

2. Procédé selon la revendication 1, dans lequel ladite étape d'analyse comprend l'application d'une transformée de Fourier sur ledit signal de grille d'antidiffusion simulée numérisée.

**3.** Procédé selon la revendication 1, dans lequel la fréquence de la grille d'antidiffusion simulée est égale à la fréquence à laquelle le signal de ladite grille d'antidiffusion possède une amplitude maximale.

**4.** Procédé selon la revendication 1, dans lequel la fréquence de la grille d'antidiffusion simulée est égale à la fréquence réelle de ladite grille d'antidiffusion.

**5.** Procédé selon la revendication 1, dans lequel la fréquence de la grille d'antidiffusion simulée est égale à une fréquence de grille estimée.

**6.** Procédé selon la revendication 1, dans lequel lesdites fréquences parasites résultant de ladite étape d'analyse qui sont présentes de manière prédominante dans ladite représentation sous forme de signal dudit signal radiographique sont mémorisées pour être supprimées.

**7.** Procédé selon la revendication 1, dans lequel lesdites fréquences parasites sont mémorisées dans une table de conversion.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6269176 B
- EP 1505540 A